# EUROPEAN PATENT APPLICATION

(11) **EP 3 146 943 A2**
(43) Date of publication of application: **29.03.2017**
(21) Application number: 16188828.4
(22) Date of filing: 14.09.2016
(51) Int. Cl.: A61F 5/01

(54) **ORTHOPEDIC BRACE DESIGNED FOR REHABILITATION OF JOINTS OF THE HUMAN BODY AND PROVIDED WITH MEANS FOR THE ADJUSTMENT OF LOOPS FOR THE STRAPS OF THE BRACE**

(30) Priority: 25.09.2015 IT UB20153886
(71) Applicant: F.G.P. S.R.L., 37062 Villafranca Di Verona (Verona) (IT)
(72) Inventor: FERRIGOLO, Moreno, 37062 VILLAFRANCA DI VERONA (Verona) (IT); TURRINI, Alberto, 37062 VILLAFRANCA DI VERONA (Verona) (IT)
(74) Representative: Sandri, Sandro

(57) **Abstract**

An orthopedic brace designed for rehabilitation of joints of the human body, the brace comprises at least one upright associated with at least one articulated coupling, wherein a bar 21 forms a part of this at least one upright. The bar 21 comprises a plurality of aligned holes 23 and a sliding elongated element 22 comprising coupling means (25, 26) for the fixing straps of the brace. In addition, this elongated element is equipped with restraining means (32) associated with the holes (23) and with a cursor (22) that can be moved in order to move the elongated element from a blocked position to an unblocked position with respect to the bar (21). The bar (21) presents a curvature wherein the concavity has a curvature radius on the longitudinal axis, facing the inside of the brace.

## Description

### TECHNICAL FIELD

This invention concerns means for the adjustment of the points of application of the loops for the straps of orthopedic braces or other similar braces designed for the rehabilitation of joints of the human body, such as the elbow, the ankle, the shoulder or similar joints.

This solution foresees the creation of a slide which can be fitted on a straight bar of orthopedic braces or similar, in order to allow the movement of the position of the straps of the brace, for example according to the length of the limb by moving the slide.

This slide presents means of restraint for the fixing of orthoses, consisting for example of straps made from fabric or flexible or elastic material.

The system makes it possible to use various elements combined together when it is necessary to find a quick and secure positioning along a linear bar.

This invention can be applied in the medical and orthopedic industry and in particular in the production of braces in general, as well as of prostheses and orthoses mainly used in conservative, post-traumatic, re-educational and post-operative therapy.

### BACKGROUND ART

It is known that the orthopedic sector makes use of an orthopedic brace or orthosis designed to guarantee or control the hinge restraint function between the femur and the tibia, or between the humerus and the radius, but also of other articulated joint points in general, supporting stress which would otherwise be harmful for the joint.

The function of an orthosis is, in general, to guarantee the relative immobilization or limitation of a joint affected by trauma, by arthrosis, by sprained ligaments or which has undergone a surgical operation.

Another use of orthoses is for the concomitant functional rehabilitation or re-education, wherein the orthosis can be used to reduce the load on the joint and reduce the pain, or can be used for prevention in cases of osteoporosis or bone weakness.

An orthosis usually comprises a rigid or soft frame, encircling the limb, designed to guarantee adequate harnessing of the joint in order to prevent the onset of stress on the ligaments or on the synovial membranes during walking by the injured and/or convalescent subject.

Taking the knee joint into consideration, the frame of classic knee braces comprises means for constraining the brace to the femur and to the tibia in areas proximal to the knee and a structure connecting these means with a hinge positioned at the level of the knee.

To ensure that the limb has sufficient freedom of movement, the frame develops for a good distance laterally to the knee, in order to allow reciprocal oscillation between the femur and the tibia.

The means of constraint usually consist of straps fitted with means, for example Velcro^{®}, particularly suitable for adjustment of the locking tension, these straps being attached to appropriate fixed supports in order to wrap around both the femur and the tibia of the user.

One of the problems encountered with the use of these traditional orthopedic braces concerns the fact that the straps are attached to the frame of the brace by means of eyelets or couplings fixed on the uprights and may also rotate on pins in order to adjust the angle of the straps with respect to the parts on which it is fitted.

This means that the brace must be produced in various sizes according to the dimensions of the limb on which it is fitted, and from an operative point of view it is necessary to produce various models of the brace, each according to the size of the person using the brace.

In turn, the production of a plurality of different models of the brace in different sizes has a considerable impact on production costs due to the need to produce various frames and various cushioning pads, and also involves storage problems since the various sales outlets need to have several items of the various sizes, with all the consequent problems also from the stock management point of view.

Document US 2013/0253394 A1 discloses an orthopedic device having first and second elongated struts each having a strap coupling member, and a flexible strap securing to the first and second struts by the strap coupling members. A semi-rigid and resiliently bendable expander is connected to and spans a predetermined length between the struts. The expander provides two main configurations for the orthopedic device including maintaining the first and second struts in a generally flat configuration relative to one another and a curved configuration upon application of a load on the first and second struts. The expander reverts to a flat configuration upon removal of a load from the first and second struts.

Document WO 02/02038 A1 discloses an orthopaedic brace including a strut length adjustment assembly, to change the operable length of the strut for sizing the brace on a patient without the need for a special tool or cutting the strut. The adjustment assembly includes a biased adjustment mechanism that coacts with a plurality of notches in the strut to variably set/position the strut relative to the adjustment assembly, to set the strut length. Each upper and lower strut preferably includes a strut length adjustment assembly to independently set the length of each strut. The strut length adjustment assembly retains a strut and includes a strap retention mechanism that is configured to releasably engage the strap.

### DESCRIPTION OF THE INVENTION

This invention proposes to provide a system for the positioning of the fixing straps of orthopedic braces designed for the rehabilitation of joints of the human body, that can eliminate or at least reduce the problems described above.

The invention proposes in particular to provide a system for the positioning of the fixing straps of orthopedic braces designed for the rehabilitation of joints, that foresees the adjustment along the respective frame uprights, of the points of application of the straps that hold the brace in place.

This is achieved by means of a system for the adjustment and tensioning of the fixing straps of an orthopedic brace designed for rehabilitation of joints of the human body, whose features are described in the main claim.

The dependent claims of the solution according to this invention describe advantageous embodiments of the invention.

The main advantages of this solution substantially concern the possibility of limiting the number of sizes of the brace to the essential ones, for example small, medium and large, since the means of adjustment according to the invention allow the movement of the attachment points of the straps closer to or farther away from the rotation axis of the brace, thus recovering any differences in positioning of the coupling points with respect to the preset points.

According to the invention, it is basically foreseen that the coupling points of the straps to the brace are located on respective elongated elements sliding on the bars that form the uprights of the brace, and that the elongated elements can be fixed in position by the use of stop means consisting of a plurality of holes positioned on the bars and engaging with a cursor and respective stop means on the elongated element.

### DESCRIPTION OF THE DRAWINGS

Other features and advantages of the invention will become clear on reading the description given below of one embodiment, provided as a non-limiting example, with the help of the accompanying drawings, in which:
- figures 1 and 2 are schematic and prospective views of the entire adjustment unit which forms the system for positioning of the fixing straps of orthopedic braces designed for the rehabilitation of joints of the human body;

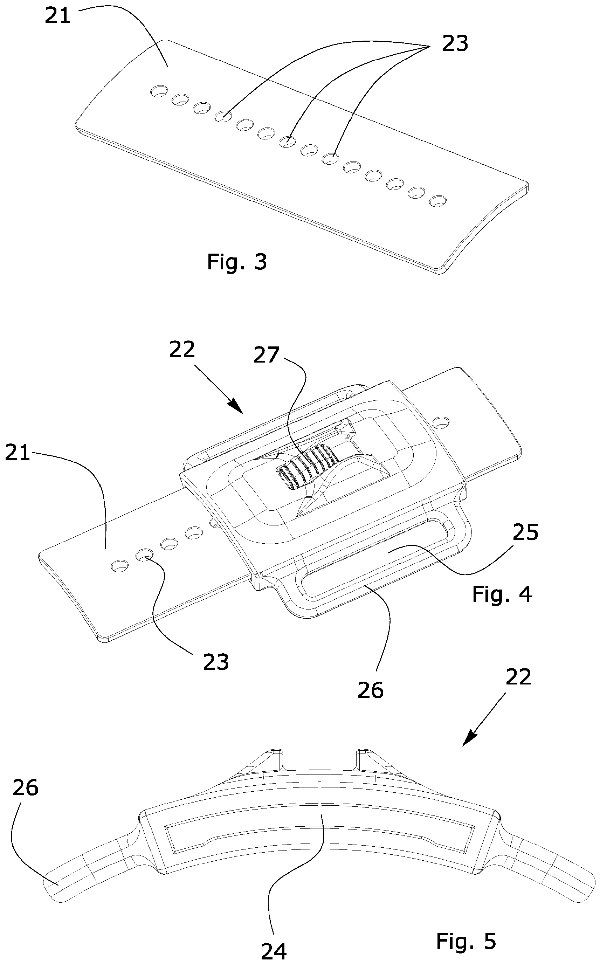

- figure 3 is a prospective view of a bar according to the invention provided with longitudinal holes that represent blocking elements for the sliding and adjustable elongated element;
- figure 4 is a view of the adjustment unit with the elongated element in another position with respect to that in Figures 1 and 2;

- Figure 5 is a schematic front view of the elongated element according to the invention;
- Figures 6 and 7 are perspective views of a elongated element without a cursor;
- figure 8 is a schematic prospective view of the elongated element according to the invention and provided with a cursor;

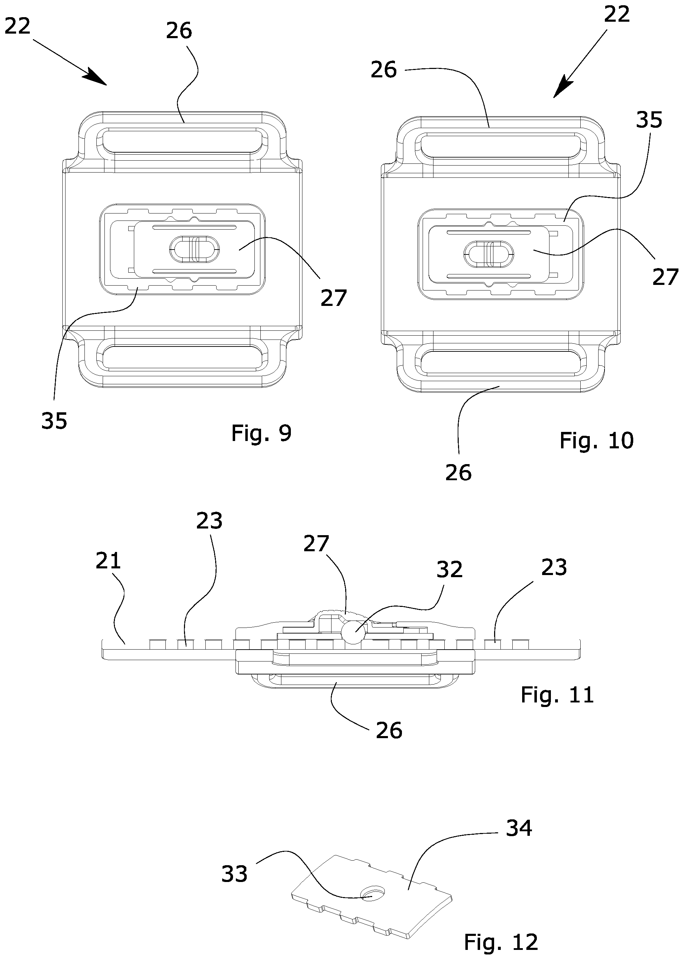

- figures 9 and 10 are schematic views of the elongated element according to the invention, shown from the inner side with the cursor moved from a release position to a blocking position;
- figure 11 is an overall vertical cross-section view of the guide and cursor assembly;
- figure 12 is a detail of the intermediate plate element;
- figure 13 shows a schematic view of a detail of figure 11 and in particular of the guide and cursor assembly with the respective ball type stop means;
- figures 14 and 15 are schematic cross-section views of the elongated element mounted on the cursor in the uncoupled and coupled positions, that is to say in the release and blocking positions.

### DESCRIPTION OF AN EMBODIMENT OF THE INVENTION

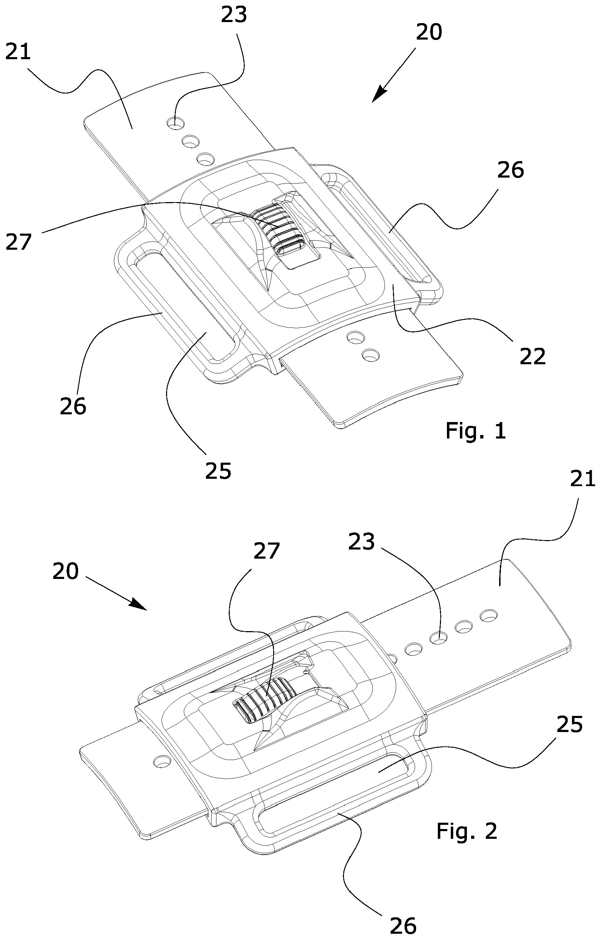

Referring first of all to figures 1 and 2, the numeral 20 denotes in its entirety the assembly of a bar 21 with a elongated element 22 which form the system for the adjustment and tensioning of the fixing straps of an orthopedic brace designed for the rehabilitation of joints of the human body.

The bar 21, which forms part of the uprights connected for example to the traditional articulated couplings for orthopedic orthoses or similar, consists of an elongated body made from rigid material, and presents a curvature with the concave side on the inside of the brace and thus a convex side facing the outside.

The longitudinal curvature of the bar 21 makes it sturdier in order to prevent bending.

This bar presents a plurality of aligned holes 23 along the centre line at a predetermined distance from each other, making it possible to block the elongated element 22 in the required position along the bas, as will be seen below.

It should be noted that the dimensions of the bar, and in particular the length, and the number of and distance between the holes 23 can be varied according to requirements.

The elongated element 22 consists of a body with a substantially four-sided shape, curved according to the curvature of the bar 21 and with a thickness that allows it to be provided internally with a through-housing 24 that can accommodate the bar 21.

The two opposite sides of the elongated element 22, parallel to the longitudinal axis of the bar, are provided with slots 25, bordered by respective loops 26, for insertion of the straps or other means for attaching the brace to the limb of the patient on which the brace is fitted.

The elongated element 22, which is free to move along the bar 21, is provided with restraining means which allow it to be placed in a blocked condition on the bar 21.

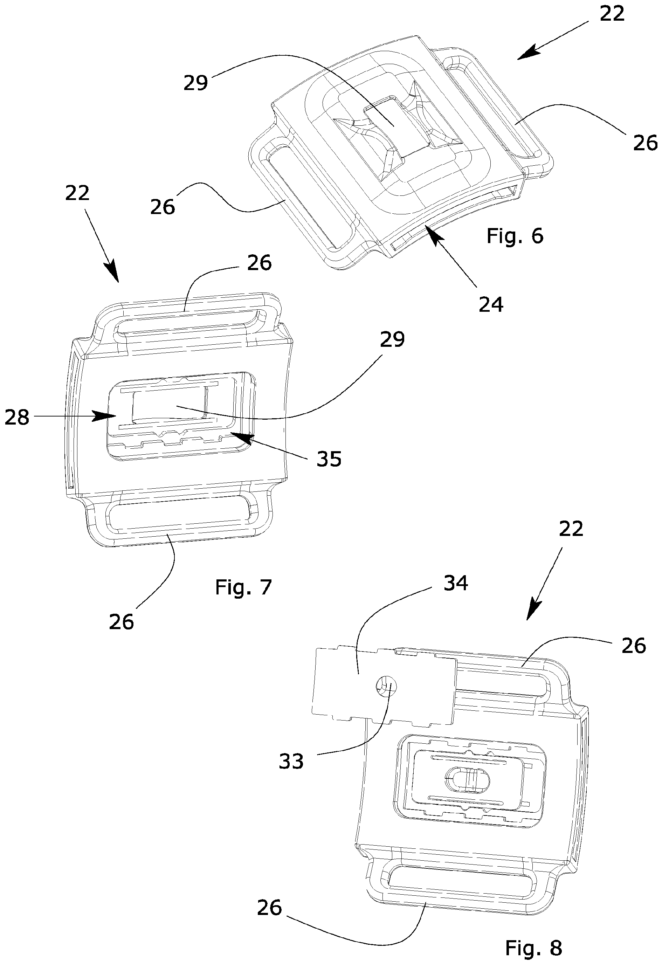

These means consist of a cursor 27, positioned inside a cavity 28 of the elongated element facing the through-housing 24, and protruding with a control piece through an opening 29 (figs. 6 and 7) in the outer surface of the elongated element 22.

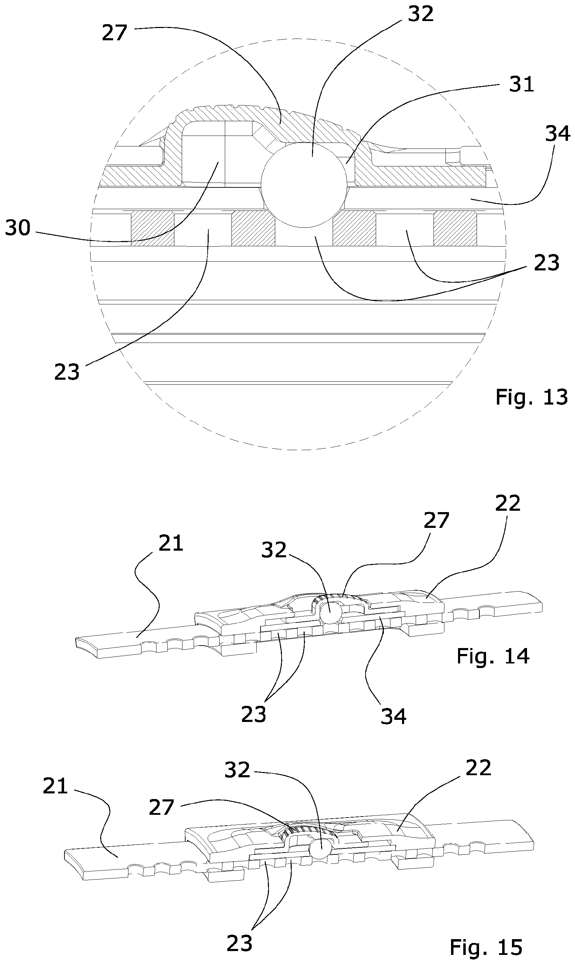

As can be seen in figure 13, the part of the cursor 27 protruding externally, that is to say the part protruding through the opening 29 and which is equipped with knurling to allow finger gripping, is hollow and the housing formed by this cavity is divided into two communicating chambers 30 and 31 in which a sphere 32 is inserted and is free to move. In particular, the chamber 30 has a greater internal volume than the chamber 31.

The sphere 32 communicates with the holes 23 in the bar 21, protruding from a hole 33 (fig. 8) in an intermediate restraining plate 34 inserted and fixed inside a housing 35 in the elongated element 22 by means of the coupling teeth with which it is equipped.

This means that the cursor 27 can be moved from a blocking position of the elongated element 22 wherein the sphere 32, pushed by the walls of the chamber 31, intercepts one of the holes 23 in the bar, and is blocked in the hole, to the condition wherein the sphere is moved into the larger chamber 30 allowing it to be released from the hole 23 into which it was inserted.

It should be noted that each hole 23, together with the hole 33 in the intermediate plate 34, has a slight flare which facilitates the insertion and restraining of the sphere 32.

From an operative point of view, the contrast to prevent the sphere from exiting the cradle formed by the hole in the bar and the hole in the intermediate plate is represented by the smaller sized chamber 31.

By moving the cursor, the sphere is released from its restraint in the hole in which it was inserted since it is moved into the larger chamber 30, and is in the blocked position.

The invention as described above refers to a preferred embodiment. It is nevertheless clear that the invention is susceptible to numerous variations falling within the scope of the disclosure, in the context of technical equivalents.

The above description refers to an embodiment of a brace for the knee, but the same system can be applied to orthoses for other joints of the human body.

## Claims

1. An orthopedic brace designed for rehabilitation of joints of the human body, the brace comprising at least one upright associated with at least one articulated joint, wherein a bar (21) forms a part of this at least one upright, said bar (21) comprising a plurality of aligned holes (23) and a sliding elongated element (22) comprising coupling means (25, 26) for the fixing straps of the brace, said elongated element being equipped with restraining means (32) associated with said holes (23) and with a cursor (27) connected to said elongated element (22) and which can be moved in order to move the elongated element from a blocked condition to an unblocked condition with respect to said bar (21), **characterised in that** said bar (21) has a curvature with a concave part with the curvature radius along the longitudinal axis, facing the inside of the brace.

2. An orthopedic brace according to claim 1, **characterised in that** said elongated element (22) consists of a body curved according to the curvature of the bar (21) and with a thickness that allows it to be provided internally with an open through-housing (24) that can accommodate the bar (21).

3. An orthopedic brace according to either of previous claims, **characterised in that** the two opposite sides of the elongated element (22) are provided with slots (25), bordered by respective loops (26), for insertion of the straps or other means for attaching the brace to the limb of the patient on which the brace is fitted.

4. An orthopedic brace according to any on of the preceding claims, **characterised in that** the cursor (27) is positioned inside a cavity (28) of the elongated element facing the through-housing (24), and protruding with a control piece through an opening (29) in the outer surface of the elongated element (22).

5. An orthopedic brace according to claim 4, **characterised in that** the part of the cursor (27) protruding externally, that is to say the part protruding through the opening (29), is equipped with knurling to allow finger gripping and is hollow, the housing formed by this cavity being divided into a first (30) and a second (31) communicating chamber with different volumes in which a sphere (32) is inserted and is free to move.

6. An orthopedic brace according to claim 5, **characterised in that** the first chamber (30) has a larger internal volume than the second chamber (31).

7. An orthopedic brace according to claim 5 or 6, **characterised in that** the sphere (32) communicates with the holes (23) in the bar (21), protruding from a hole (33) in an intermediate restraining plate (34) inserted and fixed inside a housing (35) in the elongated element (22) by means of the coupling teeth with which it is equipped.

8. An orthopedic brace according to any of the claims from 5 to 7, **characterised in that** the cursor (27) can be moved from a blocking position of the elongated element (22) in which the sphere (32), pushed by the walls of the chamber (31), intercepts one of the holes (23) in the bar, and is blocked in the hole, to the condition wherein the sphere is moved into the larger chamber (30) allowing it to be released from the hole (23) into which it was inserted.
